# EUROPÄISCHE PATENTANMELDUNG

(11) **EP 3 611 734 A1**
(43) Veröffentlichungstag der Anmeldung: **19.02.2020**
(21) Anmeldenummer: 18188666.4
(22) Anmeldetag: 13.08.2018
(51) Int. Cl.: G16H 40/67, G16H 40/20

(54) **STEUEREINHEIT UND STEUERVERFAHREN ZUR STEUERUNG EINER AUSGABEEINHEIT VON INFORMATIONEN IM RAHMEN DER MEDIZINISCHEN DIAGNOSTIK UND THERAPIE**

(71) Anmelder: Siemens Healthcare GmbH, 91052 Erlangen (DE)
(72) Erfinder: Hörnig, Mathias, 91052 Erlangen (DE)

(57) **Zusammenfassung**

Die Erfindung betrifft ein Verfahren zur Erstellung einer Steuereinheit (1) zur Steuerung einer Ausgabeeinheit (2) von Informationen im Rahmen der medizinischen Diagnostik und Therapie, umfassend die Schritte:
- Bereitstellung einer Lern-Rechenvorrichtung (4), wobei die Lern-Rechenvorrichtung (4) mittels eines Algorithmus dazu ausgestaltet ist, gesprochene Wörter zu erkennen,
- Bereitstellung einer untrainierten Steuereinheit (1a) auf oder an der Lern-Rechenvorrichtung (4), welche dazu ausgestaltet ist, mittels maschinellem Lernen trainiert zu werden,
- Bereitstellung einer Anzahl von Sprachaufnahmen (S), welche die Kommunikation während einer medizinischen Prozedur umfassen, wobei die Sprachaufnahmen (S) vergleichbare medizinische Prozeduren betreffen,
- Sprachanalyse der Sprachaufnahmen (S),
- Trainieren der Steuereinheit (1) gemäß einem Prinzip des maschinellen Lernens basierend auf der Sprachanalyse der Sprachaufnahmen (S).

Die Erfindung betrifft des Weiteren eine Steuereinheit und ein Steuerverfahren zur Steuerung einer Ausgabeeinheit von Informationen im Rahmen der medizinischen Diagnostik und Therapie, sowie ein Informationssystem zur Ausgabe von Informationen im Rahmen der medizinischen Diagnostik und Therapie.

## Beschreibung

Die Erfindung betrifft eine Steuereinheit und ein Steuerverfahren zur Steuerung einer Ausgabeeinheit von Informationen im Rahmen der medizinischen Diagnostik und Therapie, sowie ein Verfahren zur Erstellung einer solchen Steuereinheit und ein Informationssystem zur Ausgabe von Informationen im Rahmen der medizinischen Diagnostik und Therapie.

Klinische Workflows und klinische Prozeduren folgen medizinischen Standards, die sich über viele Jahre etabliert haben und sich permanent verbessern. Insbesondere sind diese Verbesserungen durch neue, schnellere, genauere Bildgebungsverfahren getrieben, beispielsweise 3D Verfahren.

Im Rahmen einer Optimierung und Automatisierung klinischer Workflows, beispielsweise bei chirurgischen Eingriffen, ist jedoch nicht nur die verwendete Technik, sondern auch der Aspekt der Kommunikation sehr wichtig. Derzeit kommt der Überwachung und Auswertung von diesbezüglicher Kommunikation in der klinischen Routine seit vielen Jahren ein steigendes Interesse zu.

Ein Problem für eine effektive Optimierung und Automatisierung klinischer Workflows jenseits rein technischer Neuerungen ist ein genaues Verständnis der jeweiligen Prozeduren. Dazu gehört insbesondere die dabei erfolgte Kommunikation, die ein wesentliches Mittel der im Untersuchungsraum anwesenden Personen (MTAs, Ärzte, Patienten) ist. Diese Kommunikation hat Auswirkungen auf den Untersuchungsablauf und dessen Dauer, die Arbeitsaufteilung, die Positionierung im Untersuchungsraum und weitere Aspekte.

Erste Ansätze in dieser Hinsicht betrafen eine Überwachung der einseitigen Kommunikation vom Personal zum Patienten, spätere Ansätze eine generelle Überwachung z.B. zu Dokumentationszwecken.

Unklar ist in dieser Hinsicht, inwieweit sich die Kommunikation im Untersuchungsraum geändert hat und von Prozedur zu Prozedur (Team zu Team) abweicht. Ferner ist unklar, inwieweit die Kommunikation für eine Routine optimiert werden kann. Des Weiteren ist nicht bekannt, wie viele Kommandos notwendig sind, und welche überflüssig oder gar destruktiv sein können.

Generell wird bei heutigen Systeme und Untersuchungen die Sprache und Kommunikation bei einem klinischen Workflow nicht ausreichend erfasst und genutzt.

Es ist eine Aufgabe der vorliegenden Erfindung, ein alternatives, komfortableres Steuerverfahren sowie eine entsprechende Steuervorrichtung zur Steuerung einer Ausgabeeinheit von Informationen im Rahmen der medizinischen Diagnostik und Therapie anzugeben, mit dem die oben beschriebenen Nachteile vermieden werden und insbesondere diejenige Kommunikation ermittelt wird (Menge und Art), die einen Workflow verbessern kann.

Diese Aufgabe wird durch ein Verfahren zur Erstellung einer Steuereinheit gemäß Patentanspruch 1, eine Steuereinheit gemäß Patentanspruch 5, ein Steuerverfahren gemäß Patentanspruch 6 und ein Informationssystem gemäß Patentanspruch 13 gelöst.

Das erfindungsgemäße Verfahren dient zur Erstellung (zum "Trainieren") einer Steuereinheit, die insbesondere zur Steuerung einer Ausgabeeinheit von Informationen im Rahmen der medizinischen Diagnostik und Therapie geeignet ist. Das Verfahren umfasst die folgenden Schritte:
- Bereitstellung einer Lern-Rechenvorrichtung
   Es wird eine Lern-Rechenvorrichtung bereitgestellt. Diese Lern-Rechenvorrichtung ist mittels eines Algorithmus dazu ausgestaltet, gesprochene Wörter zu erkennen. Dies bedeutet, dass gesprochene Wörter in datentechnische, "computerverständliche" Objekte konvertiert werden. Eine solche Erkennung und Konvertierung von Sprache ist dem Fachmann bekannt.
- Bereitstellung einer untrainierten Steuereinheit
   Es wird eine untrainierten Steuereinheit auf oder an der Lern-Rechenvorrichtung bereitgestellt. Diese untrainierte Steuereinheit ist dazu ausgestaltet, mittels maschinellem Lernen trainiert zu werden. "Untrainiert" meint in diesem Zusammenhang, dass die Steuereinheit völlig untrainiert sein kann oder nicht optimal trainiert sein kann. Die Steuereinheit kann einen trainierbaren Algorithmus umfassen oder aber eine trainierbare Hardware z.B. ein Field Programmable Gate Array (FPGA).
- Bereitstellung von Sprachaufnahmen
   Es wird eine Anzahl von Sprachaufnahmen bereitgestellt. Diese Sprachaufnahmen umfassen die Kommunikation während einer medizinischen Prozedur. Eine solche medizinische Prozedur stellt beispielsweise eine Diagnose, eine Operation oder eine Therapie dar. Beispielsweise wurde eine Reihe von Herzoperationen akustisch aufgenommen und diese Aufnahmen werden als Sprachaufnahmen zur Verfügung gestellt. Beispielsweise können die Sprachaufnahmen über ein RIS (Radiologieinformationssystem) oder ein PACS (Picture Archiving and Communication System = Bildarchivierungs- und Kommunikationssystem) zur Verfügung gestellt werden.

Die Sprachaufnahmen betreffen vergleichbare medizinische Prozeduren. Dies bedeutet, dass die Sprachaufnahmen z.B. Herzoperationen betreffen oder z.B. Diagnosen zu vergleichbaren Krankheitsbildern betreffen. Diese Vergleichbarkeit ist ein wichtiger Punkt, da sich die Kommunikation innerhalb unterschiedlicher Workflows unterscheidet. Beispielsweise werden ganz andere Worte, Befehle und Erwiderungen bei einer Lebertransplantation verwendet als bei einer Operation am Herzen. Eine Untersuchung des Gehirns verläuft anders als eine Untersuchung des motorischen Apparats. Zusammenfassend kann gesagt werden, dass eine Vergleichbarkeit vorzugsweise dann gegeben ist, wenn die gleiche medizinische Prozedur (z.B. Operation, Untersuchung, Bildaufnahme, etc.) an dem gleichen Körperbereich vorgenommen wird.

### - Sprachanalyse

Es erfolgt nun eine Sprachanalyse der Sprachaufnahmen. Diese Sprachanalyse erfolgt insbesondere prozedurabhängig, wobei eine prozedurabhängige Bedeutung bzw. Anwendung von Begriffen vorausgesetzt und angewandt wird. Die Sprachanalyse wird bevorzugt direkt von der Lern-Rechenvorrichtung durchgeführt.

### - Trainieren

Die Steuereinheit wird gemäß einem Prinzip des maschinellen Lernens basierend auf der Sprachanalyse der Sprachaufnahmen trainiert.

Die erfindungsgemäße Steuereinheit zur Steuerung einer Ausgabeeinheit von Informationen im Rahmen der medizinischen Diagnostik und Therapie ist mit dem erfindungsgemäßen Verfahren hergestellt. Es handelt sich dabei um eine trainierte Steuereinheit, die mit dem erfindungsgemäßen verfahren trainiert worden ist.

Das erfindungsgemäße Steuerverfahren dient zur Steuerung insbesondere einer Ausgabeeinheit von Informationen im Rahmen der medizinischen Diagnostik und Therapie. Insbesondere dient das Steuerverfahren für eine prozedurabhängige Spracherfassung und -analyse in der medizinischen Diagnostik und Therapie in einem Untersuchungsraum. Es wird angemerkt, dass Ausführungen, welche Besonderheiten von Komponenten des vorangehend beschriebenen Verfahrens betreffen, vorzugsweise auch für die entsprechenden Komponenten des Steuerverfahrens gelten und umgekehrt. Das Steuerverfahren umfasst die Schritte:
- Bereitstellung einer erfindungsgemäßen (trainierten) Steuereinheit oder trainieren einer Steuereinheit gemäß einem erfindungsgemäßen Verfahren.
- Erstellung einer Sprachaufnahme während einer medizinischen Prozedur. Dies kann in einem einfachen Fall dadurch geschehen, dass eine medizinische Prozedur akustisch (z.B. durch Mikrofone) während des Ablaufs in einem Untersuchungsraum aufgenommen wird. Die Steuereinheit muss dabei selbstverständlich auf diese Prozedur trainiert worden sein. Eine Steuereinheit, die mit Sprachaufnahmen einer Hüftoperation trainiert worden ist, kann z.B. nicht effektiv bei einer Herzoperation verwendet werden.
- Verarbeitung der Sprachaufnahme mittels der Steuereinheit während der Fortführung der Erstellung der Sprachaufnahme. Dies bedeutet, dass das Steuerverfahren während der medizinischen Prozedur angewandt wird. Es wird ein Teil der Kommunikation während der Prozedur aufgenommen, verarbeitet und dann der nächste Teil aufgenommen und verarbeitet.

In diesem Rahmen ermittelt die Steuereinheit Schlagworte in der Sprachaufnahme und erzeugt Ergebnis-Ausgabedaten basierend auf den Schlagworten. Es können dabei einzelne Schlagworte verwendet werden oder Schlagwortfolgen (bzw. Kombinationen aus Schlagworten). Im Grunde wurde dieses Prinzip vorzugsweise auch während des Trainings der Steuereinheit angewendet. Hier wurde die Ermittlung von Schlagworten von der Lern-Rechenvorrichtung durchgeführt und die Steuereinheit auf die Erkennung dieser Schlagworte trainiert. Gleichwohl ermittelte die Lern-Rechenvorrichtung, das Ergebnis, welches auf diese Schlagworte folgte und dieses Ergebnis wird während des Trainings der Steuereinheit in Form von Ergebnis-Ausgabedaten mit dem jeweiligen Schlagwort verknüpft.

- Steuerung der Ausgabeeinheit mit den Ergebnis-Ausgabedaten. Das Ergebnis wird hierbei bevorzugt akustisch über Sprachausgaben oder optisch, z.B. durch Schrift, Bilder oder Filme Ausgegeben. Die Ausgabe kann auf Bildschirmen oder über Lautsprecher als Ausgabeeinheit erfolgen. Die Ausgabeeinheit kann dabei direkt angesteuert werden (z.B. ein Lautsprecher in einem Operationsraum) aber auch über das Internet gesteuert werden.

Ein erfindungsgemäßes Informationssystem zur Ausgabe von Informationen im Rahmen der medizinischen Diagnostik und Therapie umfasst eine erfindungsgemäße Steuereinheit, eine Ausgabeeinheit und ein Sprachaufnahmesystem, wobei das Informationssystem zur Ausführung eines erfindungsgemäßen Steuerverfahrens ausgestaltet ist.

Eine erfindungsgemäße Lern-Rechenvorrichtung umfasst einen Prozessor und einen Datenspeicher mit Instruktionen, welche dem Prozessor bei ihrer Ausführung ermöglichen:
- der Lern-Rechenvorrichtung bereitgestellte Sprachaufnahmen zu erfassen,
- eine Analyse der Sprachaufnahmen durchzuführen (insbesondere gesprochene Worte in den Sprachaufnahmen als Objekte zu erkennen),
- ggf. Schlagworte aus den erkannten Worten zu identifizieren und
- eine Steuereinheit nach einem erfindungsgemäßen Verfahren basierend auf der Analyse der Sprachaufnahmen zu trainieren.

Ein Großteil der zuvor genannten Komponenten des Informationssystems bzw. die Steuereinheit, können ganz oder teilweise in Form von Softwaremodulen in einem Prozessor eines entsprechenden Informationssystems realisiert werden. Eine weitgehend softwaremäßige Realisierung hat den Vorteil, dass auch schon bisher verwendete Vorrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung eines Informationssystems ladbar ist, mit Programmabschnitten, um alle Schritte des erfindungsgemäßen Verfahrens auszuführen, wenn das Programm in dem Rechensystem bzw. dem Informationssystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zum Informationssystem und/oder zur Speicherung an oder in dem Rechensystem bzw. dem Informationssystem kann ein computerlesbares Medium, beispielsweise ein Memorystick, eine Festplatte oder ein sonstiger transportabler oder fest eingebauter Datenträger dienen, auf welchem die von einem Rechensystem bzw. einem Informationssystem einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Bevorzugt ist daher auch ein Informationssystem in Form eines Computerprogrammprodukts mit einem Computerprogramm, welches direkt in eine Speichereinrichtung des Informationssystems ladbar ist, mit Programmabschnitten, um alle Schritte des erfindungsgemäßen Identifikations-Verfahrens auszuführen, wenn das Computerprogramm in dem Informationssystem ausgeführt wird.

Bevorzugt ist ein Informationssystem oder eine Ausführungsform des Verfahrens oder des Steuerverfahrens in Form eines computerlesbaren Mediums, auf welchem von einer Rechnereinheit einlesbare und ausführbare Programmabschnitte gespeichert sind, um alle Schritte eines erfindungsgemäßen Verfahrens bzw. Steuerverfahrens auszuführen, wenn die Programmabschnitte von der Rechnereinheit ausgeführt werden. Die Identifikationseinheit kann in Form dieses computerlesbaren Mediums auch als Hardware vorliegen, z.B. als programmierter FPGA oder EPROM.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Das Verfahren kann auch Elemente des "Cloud Computing" (in Deutsch etwa: Berechnung in einer "Datenwolke") umfassen. Beim "Cloud Computing" wird eine IT-Infrastruktur, z.B. Speicherplatz oder Rechenleistung und/oder eine Anwendungssoftware über ein Netzwerk zur Verfügung gestellt. Die Kommunikation zwischen dem Anwender und der "Cloud" erfolgt dabei mittels Datenschnittstellen und/oder Datenübertragungsprotokollen.

Im Rahmen des "Cloud Computings" erfolgt bei einer bevorzugten Ausführungsform des erfindungsgemäßen Verfahrens eine Bereitstellung von Daten über einen Datenkanal (z.B. ein Netzwerk) an eine "Cloud". Diese "Cloud" umfasst ein (Remote-) Rechensystem, z.B. einen Computercluster, das in der Regel nicht den lokalen Rechner des Benutzers umfasst. Diese Cloud kann insbesondere durch die medizinische Einrichtung, die auch die medizintechnischen Systeme bereitstellt, zur Verfügung gestellt werden. Insbesondere werden die Daten der Bildaufnahme über ein RIS oder PACS an ein (Remote-) Rechnersystem (die "Cloud") gesendet. Bevorzugt stellen das Rechensystem der "Cloud", das Netzwerk sowie das medizintechnische System (insbesondere das oben beschriebene Informationssystem) einen Verbund im datentechnischen Sinne dar. Das Verfahren kann dabei mittels einer Befehlskonstellation in dem Netzwerk realisiert werden. Die in der Cloud berechneten Daten oder Ergebnisse werden bevorzugt wieder über einen Datenkanal (z.B. über ein Netzwerk) zu dem lokalen Rechner des Anwenders gesendet. Beispielsweise werden die Daten der bereitgestellten Bildaufnahme durch ein Rechnersystem eines Krankenhauses gemäß des erfindungsgemäßen Verfahrens bearbeitet und die Ergebnisse dieser Bearbeitung wieder durch ein RIS oder PACS an den Anwender zurückgesandt.

Im Rahmen einer bevorzugten Ausführungsform der erfindungsgemäßen Vorrichtung sind die für die Erfindung relevanten Komponenten auf Seiten der "Cloud" vorhanden. Ein bevorzugtes System umfasst zusätzlich zu einer solchen Vorrichtung noch eine lokale Recheneinheit, die über einen Datenkanal (z.B. ein Netzwerk, insbesondere als RIS oder PACS ausgestaltet) mit der Vorrichtung verbunden ist. Die lokale Rechnereinheit umfasst zumindest eine Datenempfangsschnittstelle, um Daten zu empfangen. Zudem ist es bevorzugt, wenn der lokale Rechner zudem eine Sendeschnittstelle aufweist, um Daten an die Vorrichtung zu senden.

Bei einem besonders bevorzugten Ausführungsbeispiel des Verfahrens erfasst die Sprachanalyse der Sprachaufnahmen gezielt Schlagworte der Sprachaufnahmen, insbesondere deren Wiederholungen und zeitliche Abfolgen. Es können dabei einzelne Schlagworte betrachtet werden oder Schlagwortfolgen (bzw. Kombinationen aus Schlagworten).

Bevorzugt erfolgt die Sprachanalyse pro Akteur. Es wird dabei für jede Person eine separate Sprachanalyse durchgeführt und mit der Funktion dieser Person verbunden. Beispielsweise erfolgt eine Sprachanalyse für den Arzt, eine Sprachanalyse für eine unterstützende Person (z.B. einen Pfleger oder einen Techniker) und eine Sprachanalyse für den Patienten.

Bevorzugt wird die Steuereinheit für ein erfasstes Schlagwort oder eine mit diesem Schlagwort gekoppelten Aktion auf eine Folgeaktion (bzw. auf eine proaktive Unterstützung einer Folgeaktion) trainiert. Dabei umfasst die Folgeaktion (bzw. eine proaktive Unterstützung der Folgeaktion) bevorzugt eine Geräteadaption, wie z.B. eine mechanische Positionierung, insbesondere eine Feinjustierung, eine Display-Folge-Handlungsanzeige für das Personal oder den Patienten oder eine Anzeige von Röntgenaufnahmen (bzw. Röntgenaufnahmesequenzen).

Bevorzugt wird die Sprachanalyse der Sprachaufnahmen mit einer Handlungsanalyse und/oder Aktionsanalyse basierend auf Filmaufnahmen der medizinischen Prozedur gekoppelt. Dabei werden bevorzugt die Zeitpunkte der Schlagworte, also die Zeiten zu denen die Schlagworte gesagt werden, gezielt mit Aktionen der Akteure am System und/oder im Raum aus den Filmaufnahmen datentechnisch verknüpft. Auf diese Weise können Aktionen mit Schlagwörtern gezielt korreliert werden.

Bevorzugt erfolgt eine vergleichende Sprachanalyse einer Vielzahl gleicher Prozeduren (z.B. einer Hüft-OP, einer Herzoperation, einer Diagnose). Diese wird dann als Optimierung des Trainings der Steuereinheit genutzt. Bevorzugt erfolgt eine vergleichende Sprachanalyse bezüglich der Prozedurdauer, der Vielzahl und Anzahl von Schlagwörtern, der Dauer der Sprachkommunikation und entsprechendem.

Bevorzugt wird die Sprachaufnahme durch Erfassung der Kommunikation in einem Untersuchungsraum erstellt, vorzugsweise über eine Anzahl Mikrofone. Dies sind bevorzugt deckengehängte Mikrofone und/oder an einem Gerät befestigte Mikrofone und/oder in einem Tisch integrierte Mikrofone.

Die Sprachaufnahme (also die aufgezeichnete Kommunikation im Untersuchungsraum) wird bevorzugt hinsichtlich der besten Erfassung durch die verschiedenen Mikrofone analysiert, z.B. wird ein Tischmikrofon für einen am Tisch befindlichen Operateur an Stelle eines im Raum befindlichen Deckenmikrofons genutzt, da die Sprachqualität des Tischmikrofons in diesem Fall besser ist.

Dabei erfolgt vorzugsweise zusätzlich auch eine optische Erfassung von Bereichen des Untersuchungsraumes, bevorzugt durch eine Anzahl Kameras. Als Kameras können besonders bevorzugt eine Anzahl 3D-Kameras verwendet werden.

Bevorzugt werden dabei automatisch Ergebnis-Ausgabedaten basierend auf Schlagworten gekoppelt mit der optischen Erfassung (durch die Kameraüberwachung) generiert. Dies geschieht insbesondere für dedizierte Kontrollschritte. Beispielsweise wird ermittelt, ob ein Operationsinstrument für eine Prozedur fehlt oder ein OP-Instrument im Vergleich zum Prozedurstart fehlt.

Bevorzugt ist für ein erfasstes Schlagwort oder eine mit diesem Schlagwort gekoppelte Aktion in den Ergebnis-Ausgabedaten eine von der Steuereinheit gelernte Folgeaktionen enthalten (bzw. eine proaktive Unterstützung einer Folgeaktion). Dabei umfasst die Folgeaktion (bzw. eine proaktive Unterstützung der Folgeaktion) bevorzugt eine Geräteadaption, wie z.B. eine mechanische Positionierung, insbesondere eine Feinjustierung, eine Display-Folge-Handlungsanzeige für das Personal oder den Patienten oder eine Anzeige von Röntgenaufnahmen (bzw. Röntgenaufnahmesequenzen).

Bevorzugt ist die Steuereinheit bevorzugt dazu ausgelegt, dass Personen während der medizinischen Prozedur (z.B. in einem Untersuchungsraum) über Mikrofone Fragen stellen können und die Steuereinheit aus Schlagworten in den Fragen als Ergebnis-Ausgabedaten (vorgegebene) Antworten generiert. Diese Antworten können beispielsweise mit vorgegebenen Antworten gekoppelt sein, so dass bei der Konstellation der Schlagworte "Information zu dem Patenten" Ergebnis-Ausgabedaten ausgegeben werden, welche die Ausgabeeinheit dermaßen steuern, dass diese die Krankenakte ausgibt.

Bevorzugt umfasst die Ausgabeeinheit Lautsprecher im Untersuchungsraum, z.B. integriert in einem medizinischen System, einem medizintechnischen Gerät und/oder in einem Tisch. Dabei sind die auf diese Weise ausgegebene Ergebnis-Ausgabedaten so gestaltet, dass sie eine synthetische Sprachausgabe über die Lautsprecher bewirken. So können die Lautsprecher gezielt zur Sprachübertragung bzw. Sprachausgabe genutzt werden. Die Steuereinheit ist dabei bevorzugt so ausgestaltet, dass sie die Ergebnis-Ausgabedaten so generiert, dass einem Akteur im Untersuchungsraum spezifische Hinweise über einen ihm nahen Lautsprecher ausgegeben werden.

Bevorzugt ist die Steuereinheit so ausgestaltet, dass bei ihr per Sprachsteuerung (mittels Schlagwörtern) Hilfe angefordert werden kann. Dabei ist die Steuereinheit bevorzugt so ausgestaltet, dass sie Ergebnis-Ausgabedaten umfassend Filmdaten für Dokumentationszwecke ausgibt. Auf diese Weise kann z.B. ein "Role Model" Film ausgegeben werden, dieses liegt bevorzugt als Filmaufzeichnung in einem Speicherbereich abgespeichert vor, und kann während der Prozedur beliebig abgerufen werden. Beispielhaft wäre auch als Kontrollfunktion die Ausgabe einer zuvor durchgeführten Aktion, z.B. einer Röntgen-Fluoroskopieszene oder der Filmausgabe einer vorherigen, erfassten (OP-)Aktion. Die Filmaufzeichnung wird bei Abruf bevorzugt automatisch in die Ergebnis-Ausgabedaten integriert.

Bevorzugt umfassen die Ergebnis-Ausgabedaten Daten für eine synthetische Sprachausgabe. Bevorzugt werden dabei anhand der erfassten Kommunikationen für dedizierte Prozeduren über die am System bzw. im Raum installierten Lautsprecher gezielt anhand von Aktionen oder zeitlicher Abfolge Schlagwörter, Sprachkommandos oder Sprachhinweise während einer medizinischen Prozedur abgespielt. Dabei ist die Steuereinheit bevorzugt so ausgestaltet, dass eine synthetische Sprachausgabe für dedizierte Akteure (z.B. einen MTA, Operateur) aktiviert werden kann. Diese Aktivierung kann automatisch, manuell oder per Schlagwort erfolgen.

Bevorzugt wird die durch die Ergebnis-Ausgabedaten bewirkte Ausgabe (z.B. eine synthetische Sprachausgabe oder ein abgespielter Film) in einer weiteren Phase während und/oder nach der Ausgabe gezielt hinsichtlich der Akzeptanz durch die Akteure analysiert. Beispielsweise wird die Wortfolge "Das war nicht hilfreich" darauf hindeuten, dass die Ausgabe nicht passend war. Dies dient der Effizienzmessung der Steuereinheit. Es wird dabei untersucht, ob die Ausgabe hilfreich war, und die Steuereinheit ggf. dadurch weiter trainiert.

Vorteile der Erfindung bestehen darin, dass erstmals der Zusammenhang zwischen Sprache und einer damit verbundener Aktion durch den Akteur hergestellt werden kann, die Kommunikation als Mittel zur Workflowoptimierung erfasst und genutzt werden kann, Die Kommunikation darüber hinaus zur Entwicklung und Optimierung synthetischer Sprachausgaben genutzt werden kann, synthetische Sprachausgaben für Hinweise und Kontrollen bei Prozeduren genutzt werden können. Dadurch können Fehler vermieden werden. Ebenso können ineffiziente Workflows und Kommunikationen erfasst und nachfolgend vermieden werden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
- Figur 1: einen Ablaufplan für einen möglichen Ablauf eines erfindungsgemäßen Verfahrens,
- Figur 2: eine grob schematische Darstellung eines bevorzugten Informationssystems,
- Figur 3: einen Ablaufplan für einen möglichen Ablauf eines erfindungsgemäßen Steuerverfahrens.

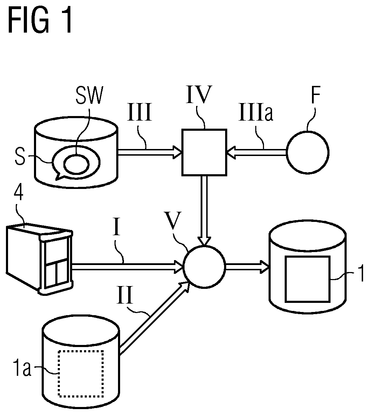

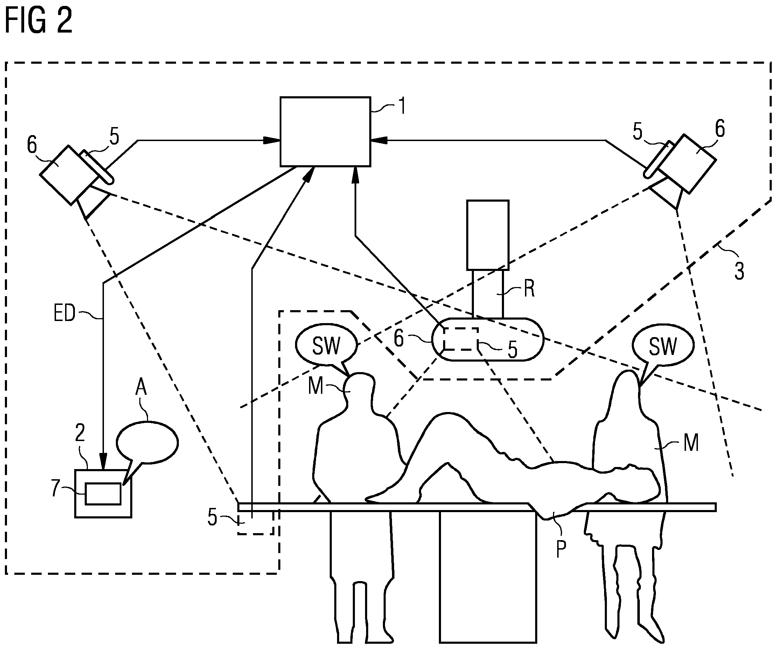

Figur 1 zeigt ein Ablaufplan für einen möglichen Ablauf eines erfindungsgemäßen Verfahrens zur Erstellung einer Steuereinheit 1 zur Steuerung einer Ausgabeeinheit 2 (s. dazu z.B. Figur 2) von Informationen im Rahmen der medizinischen Diagnostik und Therapie. Das Verfahren umfasst die folgenden Schritte:

In Schritt I wird eine Lern-Rechenvorrichtung 4 bereitgestellt. Diese Lern-Rechenvorrichtung 4 ist mittels eines Algorithmus dazu ausgestaltet, gesprochene Wörter zu erkennen und selbstverständlich auch eine Steuereinheit 1 zu trainieren. Die Lern-Rechenvorrichtung 4 kann, wie hier dargestellt, ein leistungsfähiger Computer sein, sie kann aber auch eine virtuelle Maschine in einem Rechensystem sein.

In Schritt II wird eine untrainierte Steuereinheit 1a bereitgestellt, welche dazu ausgestaltet ist, mittels maschinellem Lernen trainiert zu werden. Diese Steuereinheit 1a kann ein ungelernter Algorithmus sein, der als Software auf der Lern-Rechenvorrichtung 4 bereitgestellt wird, sie kann aber auch eine Hardwareeinheit sein, z.B. ein programmierbares Field Array (FPGA) oder ein eigenständiger Kleincomputer, der an der Lern-Rechenvorrichtung 4 (also mit der Lern-Rechenvorrichtung 4 verbunden) bereitgestellt wird.

In Schritt III wird eine Anzahl von Sprachaufnahmen S bereitgestellt, welche die Kommunikation während einer medizinischen Prozedur umfassen. Eine Sprachaufnahme ist hier als Sprechblase dargestellt, welche Informationen einer gesprochenen Kommunikation symbolisiert und hier ein Schlagwort SW umfasst. Eine Vielzahl solcher Sprachaufnahmen S werden in diesem Verfahren zum Training eingesetzt, wobei alle diese Sprachaufnahmen S vergleichbare medizinische Prozeduren, z.B. eine Hüftoperation oder eine Röntgenaufnahme des Bauchraumes betreffen.

In Schritt IV wird eine Sprachanalyse der Sprachaufnahmen S durchgeführt. Die Sprachanalyse wird bevorzugt direkt von der Lern-Rechenvorrichtung 4 durchgeführt.

Bei der Sprachanalyse der Sprachaufnahmen S werden bevorzugt gezielt Schlagworte SW der Sprachaufnahmen S erfasst, z.B. Wiederholungen und zeitliche Abfolgen der Schlagworte SW.

Es ist vorteilhaft, wenn die Sprachanalyse der Sprachaufnahmen S mit einer Handlungsanalyse bzw. Aktionsanalyse basierend auf Filmaufnahmen F der medizinischen Prozedur gekoppelt wird. Dabei werden bevorzugt die Zeitpunkte der Schlagworte SW, also diejenigen Uhrzeiten, zu denen die Schlagworte gesagt wurden, gezielt mit Aktionen der Akteure am System und/oder im Raum aus den Filmaufnahmen F datentechnisch verknüpft.

In dem optionalen Schritt IIIa werden diese Filmaufnahmen F bereitgestellt.

In Schritt V, der hier als Kreis dargestellt ist, erfolgt ein Training der untrainierten Steuereinheit 1a gemäß einem Prinzip des maschinellen Lernens basierend auf der Sprachanalyse der Sprachaufnahmen S. Dieses Training erfolgt auf der Lern-Rechenvorrichtung, die symbolisch mit dem Pfeil zum Schritt I in den Kreis hineingezogen wird.

Als Ergebnis erhält man eine trainierte Steuereinheit 1.

Figur 2 zeigt eine grob schematische Darstellung eines bevorzugten Informationssystems 3. Bei den folgenden Erläuterungen wird davon ausgegangen, dass es sich bei der dargestellten Szene um eine Röntgenuntersuchung handelt. Grundsätzlich ist das Verfahren aber für eine Vielzahl unterschiedlicher medizinischer Vorgänge ("Workflows"), wie z.B. Operationen oder Untersuchungen, vorteilhaft einsetzbar.

In eine Untersuchungsraum liegt ein Patient P unter einem Röntgengerät R auf einer Liege und erwartet eine Bildaufnahme des Unterleibs. Zwei Mediziner M stehen an der Seite des Patienten P und unterhalten sich über den Ablauf der Aufnahmeprozedur. Im Untersuchungsraum befinden sich zwei Kameras 6 an der Decke, welche den Untersuchungsraum überwachen und Filmaufnahmen F erstellen. Diese Kameras weise Mikrofone 5 auf, welche die im Untersuchungsraum stattfindenden Unterhaltung als Sprachaufnahmen S aufzeichnen. Zusätzlich befindet sich noch eine Kamera 6 zusammen mit einem Mikrofon 5 im Röntgengerät. Die Kamera 6 nimmt dabei den Patienten und ggf. dessen Reaktionen auf. Ein weiteres Mikrofon befindet sich an der Liege.

Die Sprachaufnahmen werden von der Steuereinheit 1 erfasst und mit einer Sprachanalyse ausgewertet ggf. nur diejenige mit der besten Sprachqualität. Zusätzlich werden in diesem Beispiel auch noch die mit den Kameras 6 angefertigten Filmaufnahmen F ausgewertet bzw. analysiert. Auf die bei der Sprachanalyse erkannten Schlagworte SW der Mediziner M werden von der trainierten Steuereinheit 1 Ergebnis-Ausgabedaten ED generiert und mittels dieser Ergebnis-Ausgabedaten ED eine (Sprach-)Ausgabe A mittels eines Lautsprechers 7 ausgegeben.

Beispielsweise könnte die rechts stehende Person M die links stehende Person M fragen, welche Röntgenenergie für den Patienten P eingestellt werden muss. Die links stehende Person M sagt "Bitte Krankenakte". Daraufhin erkennt die Steuereinheit das Schlagwort "Krankenakte" von der links stehenden Person M und die Schlagwortfolge "Welche Röntgenenergie" der rechts stehende Person M und sucht daraufhin nach einem entsprechenden Eintrag in der Krankenakte, erzeugt Ergebnis-Ausgabedaten ED mit einem Soundfile, welches über den Lautsprecher 7 ausgegeben wird. Die Ausgabe A könnte lauten "Patient P wurde bisher mit der Energie X keV bestrahlt".

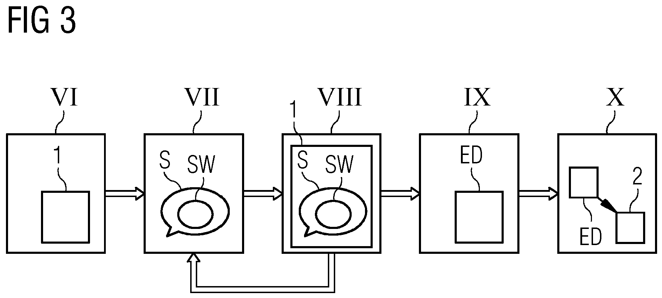

Figur 3 zeigt einen Ablaufplan für einen möglichen Ablauf eines erfindungsgemäßen Steuerverfahrens zur Steuerung einer Ausgabeeinheit 2 von Informationen im Rahmen der medizinischen Diagnostik und Therapie.

In Schritt VI erfolgt eine Bereitstellung einer trainierten Steuereinheit 1 (s. dazu z.B. Figur 1).

In Schritt VII erfolgt eine Erstellung einer Sprachaufnahme S während einer medizinischen Prozedur.

In Schritt VIII erfolgt eine Verarbeitung der Sprachaufnahme S mittels der Steuereinheit 1. Die Steuereinheit 1 ermittelt Schlagworte SW in der jeweils erstellten Sprachaufnahme S und erzeugt Ergebnis-Ausgabedaten ED basierend auf diesen Schlagworten SW.

Der zu Schritt VII rückführende Pfeil verdeutlicht, dass diese beiden Schritte fortlaufend wiederholt werden, so dass eine Fortführung der Erstellung der Sprachaufnahme S stattfindet.

In Schritt IX erfolgt eine Steuerung der Ausgabeeinheit 2 mit den Ergebnis-Ausgabedaten ED.

Es wird abschließend noch einmal darauf hingewiesen, dass es sich bei den vorhergehend detailliert beschriebenen Ausführungsformen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Erstellung einer Steuereinheit (1) zur Steuerung einer Ausgabeeinheit (2) von Informationen im Rahmen der medizinischen Diagnostik und Therapie, umfassend die Schritte:
- Bereitstellung einer Lern-Rechenvorrichtung (4), wobei die Lern-Rechenvorrichtung (4) mittels eines Algorithmus dazu ausgestaltet ist, gesprochene Wörter zu erkennen,
- Bereitstellung einer untrainierten Steuereinheit (1a) auf oder an der Lern-Rechenvorrichtung (4), welche dazu ausgestaltet ist, mittels maschinellem Lernen trainiert zu werden,
- Bereitstellung einer Anzahl von Sprachaufnahmen (S), welche die Kommunikation während einer medizinischen Prozedur umfassen, wobei die Sprachaufnahmen (S) vergleichbare medizinische Prozeduren betreffen,
- Sprachanalyse der Sprachaufnahmen (S),
- Trainieren der untrainierten Steuereinheit (1a) gemäß einem Prinzip des maschinellen Lernens basierend auf der Sprachanalyse der Sprachaufnahmen (S).

2. Verfahren nach Anspruch 1, wobei die Sprachanalyse der Sprachaufnahmen (S) gezielt Schlagworte (SW) der Sprachaufnahmen (S) erfasst, insbesondere deren Wiederholungen und zeitliche Abfolgen, und/oder wobei die Sprachanalyse pro Akteur erfolgt,
wobei bevorzugt für ein erfasstes Schlagwort (SW) oder eine mit diesem Schlagwort (SW) gekoppelte Aktion die Steuereinheit (1a) auf eine Folgeaktion trainiert wird, wobei die Folgeaktion bevorzugt eine Geräteadaption, eine Display-Folge-Handlungsanzeige für das Personal oder den Patienten oder eine Anzeige von Röntgenaufnahmen umfasst.

3. Verfahren nach einem der vorangehenden Ansprüche, wobei die Sprachanalyse der Sprachaufnahmen (S) mit einer Handlungsanalyse und/oder Aktionsanalyse basierend auf Filmaufnahmen (F) der medizinischen Prozedur gekoppelt wird,
wobei bevorzugt die Zeitpunkte der Schlagworte (SW) gezielt mit Aktionen der Akteure am System und/oder im Raum aus den Filmaufnahmen (F) datentechnisch verknüpft werden.

4. Verfahren nach einem der vorangehenden Ansprüche, wobei eine vergleichende Sprachanalyse einer Vielzahl gleicher Prozeduren erfolgt und diese als Optimierung des Trainings der Steuereinheit (1) genutzt wird, bevorzugt bezüglich Prozedurdauer, Vielzahl und Anzahl von Schlagwörtern, Dauer der Sprachkommunikation.

5. Steuereinheit (1) zur Steuerung einer Ausgabeeinheit (2) von Informationen im Rahmen der medizinischen Diagnostik und Therapie, herstellbar mit einem Verfahren nach einem der Ansprüche 1 bis 4.

6. Steuerverfahren zur Steuerung einer Ausgabeeinheit (2) von Informationen im Rahmen der medizinischen Diagnostik und Therapie,
umfassend die Schritte:
- Bereitstellung einer trainierten Steuereinheit (1) nach Anspruch 5 oder Trainieren einer untrainierten Steuereinheit (1a) gemäß einem der Ansprüche 1 bis 4,
- Erstellung einer Sprachaufnahme (S) während einer medizinischen Prozedur,
- Verarbeitung der Sprachaufnahme (S) mittels der Steuereinheit (1) während der Fortführung der Erstellung der Sprachaufnahme (S), wobei die Steuereinheit (1) Schlagworte (SW) in der Sprachaufnahme (S) ermittelt und Ergebnis-Ausgabedaten (ED) basierend auf den Schlagworten (SW) erzeugt,
- Steuerung der Ausgabeeinheit (2) mit den Ergebnis-Ausgabedaten (ED).

7. Steuerverfahren nach Anspruch 6, wobei die Sprachaufnahme (S) durch Erfassung der Kommunikation in einem Untersuchungsraum erstellt wird, vorzugsweise über eine Anzahl Mikrofone (5),
wobei die Sprachaufnahme (S) bevorzugt hinsichtlich der besten Erfassung durch die verschiedenen Mikrofone (5) analysiert wird, wobei vorzugsweise zusätzlich auch eine optische Erfassung von Bereichen des Untersuchungsraumes erfolgt, bevorzugt durch eine Anzahl Kameras (6), umfassend besonders bevorzugt eine Anzahl 3D-Kameras,
wobei bevorzugt Ergebnis-Ausgabedaten (ED) basierend auf Schlagworten (SW) gekoppelt mit der optischen Erfassung automatisch generiert werden,
und/oder wobei bevorzugt für ein erfasstes Schlagwort (SW) oder einer mit diesem Schlagwort (SW) gekoppelten Aktion in den Ergebnis-Ausgabedaten (ED) eine von der Steuereinheit (1) gelernte Folgeaktionen enthalten ist, wobei eine bevorzugte Folgeaktion eine Geräteadaption, eine Display-Folge-Handlungsanzeige für das Personal oder den Patienten oder eine Anzeige von Röntgenaufnahmen umfasst.

8. Steuerverfahren nach Anspruch 6 oder 7, wobei die Steuereinheit (1) bevorzugt dazu ausgelegt ist, dass Personen während der medizinischen Prozedur über Mikrofone (5) Fragen stellen können und die Steuereinheit (1) aus Schlagworten (SW) in den Fragen als Ergebnis-Ausgabedaten (ED) Antworten generiert.

9. Steuerverfahren nach einem der Ansprüche 6 bis 8, wobei die Ausgabeeinheit (2) Lautsprecher (7) im Untersuchungsraum umfasst, wobei die auf diese Weise ausgegebenen Ergebnis-Ausgabedaten (ED) so gestaltet sind, dass sie eine Ausgabe (A) von synthetischer Sprache über die Lautsprecher (7) bewirken und wobei die Steuereinheit (1) bevorzugt so ausgestaltet ist, dass sie die Ergebnis-Ausgabedaten (ED) so generiert, dass einem Akteur im Untersuchungsraum spezifische Hinweise über einen ihm nahen Lautsprecher (7) ausgegeben werden.

10. Steuerverfahren nach einem der Ansprüche 6 bis 9, wobei die Steuereinheit (1) so ausgestaltet ist, dass bei ihr per Sprachsteuerung Hilfe angefordert werden kann,
wobei die Steuereinheit (1) bevorzugt so ausgestaltet ist, dass sie Ergebnis-Ausgabedaten (ED) umfassend Filmdaten für Dokumentationszwecke ausgibt.

11. Steuerverfahren nach einem der Ansprüche 6 bis 10, wobei die Ergebnis-Ausgabedaten (ED) Daten für eine Ausgabe (A), bevorzugt synthetischer Sprache, umfassen,
wobei die Steuereinheit (1) bevorzugt so ausgestaltet ist, dass eine Ausgabe (A) für dedizierte Akteure aktiviert werden kann.

12. Steuerverfahren nach einem der Ansprüche 6 bis 11, wobei die durch die Ergebnis-Ausgabedaten (ED) bewirkte Ausgabe (A) in einer weiteren Phase während und/oder nach der Ausgabe (A) gezielt hinsichtlich der Akzeptanz durch die Akteure analysiert wird.

13. Informationssystem (3) zur Ausgabe von Informationen im Rahmen der medizinischen Diagnostik und Therapie umfassend eine Steuereinheit (1) gemäß Anspruch 5, eine Ausgabeeinheit (2) und ein Sprachaufnahmesystem (7), wobei das Informationssystem (3) zur Ausführung eines Steuerverfahrens nach einem der Ansprüche 6 bis 12 ausgestaltet ist.

14. Computerprogrammprodukt mit einem Computerprogramm, welches direkt in eine Speichereinrichtung eines Rechensystems (3) oder eines Informationssystems (3) zur Ausgabe von Informationen im Rahmen der medizinischen Diagnostik und Therapie, ladbar ist, mit Programmabschnitten, um alle Schritte des Steuerverfahrens nach einem der Ansprüche 6 bis 12 auszuführen, wenn das Computerprogramm in dem Rechensystem (3) oder dem Informationssystem (3) ausgeführt wird.

15. Computerlesbares Medium, auf welchem von einer Rechnereinheit einlesbare und ausführbare Programmabschnitte gespeichert sind, um alle Schritte eines Verfahrens nach einem der Ansprüche 1 bis 4 oder eines Steuerverfahrens nach einem der Ansprüche 6 bis 12 auszuführen, wenn die Programmabschnitte von der Rechnereinheit ausgeführt werden.
